# EUROPÄISCHE PATENTANMELDUNG

(11) **EP 2 110 073 A1**
(43) Veröffentlichungstag der Anmeldung: **21.10.2009**
(21) Anmeldenummer: 08154604.6
(22) Anmeldetag: 16.04.2008
(51) Int. Cl.: A61B 5/00

(54) **Quantitative und hochaufgelöste optische Tomographie**

(71) Anmelder: DKFZ Deutsches Krebsforschungszentrum, 69120 Heidelberg (DE)
(72) Erfinder: Schulz, Ralf B., Dr., 81735 München (DE); Kiessling, Fabian, 69120 Heidelberg (DE); Semmler, Wolfhard, Prof. Dr., 69118 Heidelberg (DE)
(74) Vertreter: Hörschler, Wolfram Johannes

(57) **Zusammenfassung**

Die Erfindung bezieht sich auf ein Bildgebungsverfahren zur Darstellung beschränkter Ausschnitte eines Gewebes (66), zur Ticfcnauflösung des Gewebes (66) und zur Quantifizierung mindestens einer fluoreszierenden Markersubstanz im Gewebe (66). Dem vorgeschlagenen Bildgebungsverfahren folgend werden folgende Verfahrensschritte durchlaufen: Als Anregungsquelle (20) wird ein bevorzugt monochromatischer Lichtstrahl verwendet, der über einen dichromatischcn Spiegel (26) auf ein Objekt (12) gerichtet wird. Der bevorzugt monochromatische Lichtstrahl wird innerhalb eines Scanbereiches (22)/Verfahrbereiches (24) relativ zum Objekt (12) verfahren. Eine Detektion von Emissionen (36; 54, 60) erfolgt durch eine einem Emissionsfilter (38) nachgeschaltete CCD-Kamera (40).

## Beschreibung

Die vorliegende Erfindung bezieht sich auf ein Verfahren zur quantitativen und hochauflösenden optischen Tomographie mittels eines Intravitalmikroskopes, insbesondere zur Intravitaltomographie.

### Stand der Technik

Aus der Publikation Elizabeth M. C. Hillman, David A. Boas, Anders M. Dale und Andrew K. Dunn, "Laminar optical tomography: demonstration of millimeter-scale depthresolved imaging in turbid media", OPTICS LETTERS, Vol. 29, No. 14, July 15, 2004, Seiten 1650-1652, ist ein Verfahren bekannt, welches als LOT (Laminar Optical Tomography) bezeichnet wird. Gemäß diesem Verfahren werden die Vorteile der optischen Tomographie, namentlich die Tiefenauflösung durch die Bildrekonstruktion, mit einem Mikroskop-basierten Aufbau kombiniert. Dadurch wird eine Bildaufnahme mit einer Auflösung zwischen 100 - 200 µm erreicht über eine Eindringtiefe von 0 - 2,5 mm. Das LOT-Verfahren ist primär entwickelt worden zur multispektralen Bildgebung von Ratten, bei der eine Auflösungsdynamik in verschiedenen Schichten des Gehirns bis in Tiefen von 1500 µm von steigendem Interesse für die Neurophysiologen ist. Das Verfahren von Hillman et al. wird verwendet, um zwischen arteriellem und venösem Gefäßsystem unterscheiden zu können und somit genaue Erkenntnisse über die cerebrale Hämodynamik und damit indirekt über die Sauerstoffversorgung des Gehirns zu erhalten.

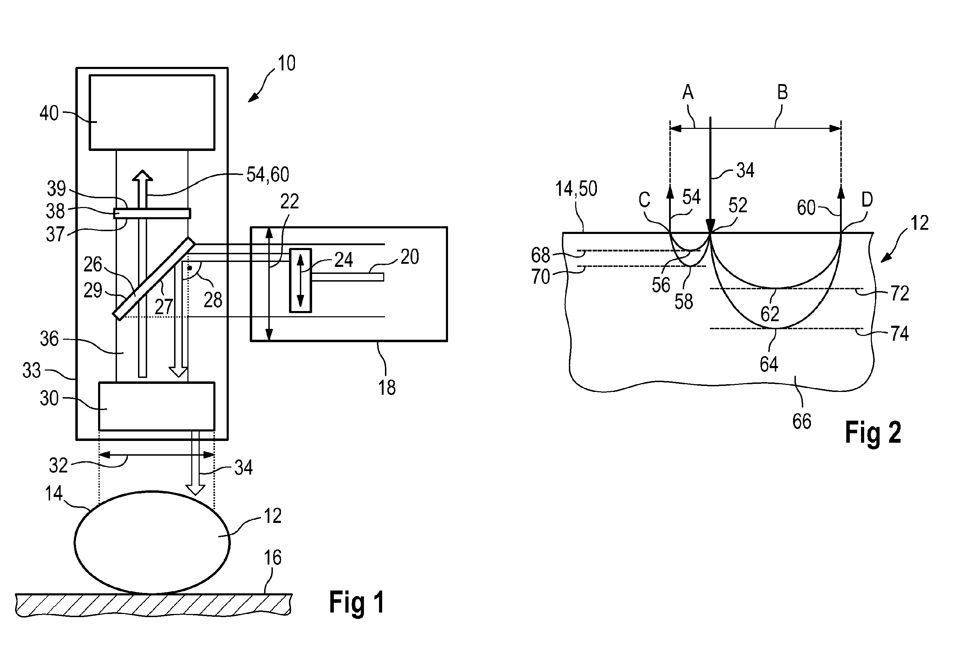

Aus der Publikation "Fibered Confocal Fluorescence Microscopy (Cell-viZio^{™}) Facilitates Extended Imaging in the Field of Microcirculation", A Comparison with Intravital Microscopy, Elisabeth Laemmel, Magalie Genet, Georges Le Goualher, Aymeric Perchant, Jean-François Le Gargasson und Eric Vicaut, Journal of Vascular Research, 2004;41: 400-411, DOI: 10.1159/000081209, ist ein Verfahren bekannt, welches in vivo mikrovaskuläre Beobachtungen gestattet. Das in dieser Publikation veröffentlichte FCFM (Fibered Confocal Fluorescence Microscopy)-Verfahren ist speziell dafür ausgelegt, in vivo in situ Beobachtungen, d. h. Bilder aufzunehmen, was dank eines Messaufbaus möglich ist, der Faserbündel und mikrooptische Elemente umfasst, die einen Durchmesser in der Größenordnung von 650 µm aufweisen. Gemäß dem Messaufbau (vergleiche Figur 1 der genannten Publikation) wird ein Intravital-Fluoreszenzmikroskop eingesetzt, welches ein konventionelles Fluoreszenzmikroskop mit 20 Objektiven einer numerischen Apertur von 0,32 aufweist. Mittels einer Xenonlichtquelle wird die Epifluoreszenzbeleuchtung vorgenommen, ferner waren ein Hitzefilter und ein Anregungsfilter vorgesehen. Bilder wurden aufgenommen mit Hilfe einer Analysesoftware, die wiederum eine CCD-Hochleistungskamera steuerten.

Zur Darstellung von Fluoreszenz existieren zahlreiche Verfahren.

Es werden mikroskopische Verfahren eingesetzt, wie zum Beispiel die Fluoreszenzmikroskopie oder die Konfokalmikroskopie. Es können auch noch weitere höherauflösende Verfahren eingesetzt werden, die sich jedoch noch im Experimentalstadium befinden. Die mikroskopischen Verfahren haben ihr Anwendungsgebiet hauptsächlich im Bereich der Analyse von Gewebeschnitten oder in der Analyse einzelner Zellen. Die Lichtstreuung in größeren Gewebebereichen ist jedoch für die Anwendung derartiger Verfahren im Rahmen der in vivo-Bildgebung ein großer Nachteil.

Die aus der Fluoreszenzmikroskopie abgeleitete Intravitalmikroskopie arbeitet auf großflächigen Arealen. Es könnte jedoch auch durchaus sinnvoll sein, ein Objektiv mit geringerer bis mittlerer Vergrößerung einzusetzen. Prinzipiell jedoch ist die konstruktive Grundlage der vorliegenden Erfindung unabhängig vom Vergrößerungsfaktor. Bei der Intravitalmikroskopie lassen sich Bereiche einer Größe von mehreren Quadratmillimetern Fläche am lebenden Tier abbilden. Die Tiefe und Quantität fluoreszierender Quellen, die einige Millimeter tief im Gewebe des lebenden Tieres lokalisiert sein können, kann jedoch im Wege der Intravitalmikroskopie nur unzureichend genau bestimmt werden. Hauptsächlich findet daher die Intravialmikroskopie in Verbindung mit Hautklappentechniken oder Haut-/Schädelfenstern Verwendung. Hautklappen oder Hautfenster sind Stellen, an denen die Haut des Versuchstiers entfernt ist, um die durch die Haut erzeugte Streuung und Schwächung zu beseitigen und direkten Einblick unter die Haut zu gewährleisten. Bei der Hautklappe wird dabei die Haut vorübergehend umgeklappt und nach Begutachtung wieder angenäht. Beim Fenster wird die Haut entfernt und durch einen Ring verhindert, dass die freigelegte Stelle wieder zuwächst. Um eine Austrocknung zu vermeiden, wird dabei meist ein glasklares, luftdichtes Material auf diese Stelle aufgebracht. Fenster und Klappen werden hauptsächlich eingesetzt, um die hohe Streuung, die sich einstellende Autofluoreszenz und die Schwächung des Signals durch die obere Hautschicht zu eliminieren.

Die Fluoreszenztomographie versucht, quantitative und räumlich aufgelöste Informationen aus der Durchleuchtung von Gewebe zu erhalten. Dabei wird der Umstand ausgenutzt, dass Gewebe für Licht im (Nah-)Infrarotbereich besonders gut durchlässig ist. Für andere Wellenlängenbereiche, wie zum Beispiel Grün, Gelb oder Rot, die aufgrund der guten Verfügbarkeit zum Beispiel fluoreszierender Proteinen in diesem Farben eine wichtige Rolle in der tierexperimentellen Forschung spielen, lässt sich die vollständige Durchleuchtung ganzer Tiere nur schwer oder gar nicht erreichen, da die Eindringtiefe stark limitiert ist. Die räumliche Auflösung der Fluoreszenztomographie liegt nur im Bereich weniger Millimeter.

Die Darstellung dieser Farbstoffe im lebenden Tier gelingt zwar durch die Verwendung einer großflächigen und intensiven Ausleuchtung sowie optimierten Filtern, wie zum Beispiel aus der Publikation "The multiple uses of fluorescent proteins to visualize cancer in-vivo", R. M. Hoffman, Nat. Rev. Canc. 5: 796-806, 2005, bekannt ist. Eine Ermittlung der räumlichen Tiefe der Emissionsquellen und der dort tatsächlich vorhandenen Farbstoffkonzentrationen ist in diesem Fall jedoch nicht möglich.

### Offenbarung der Erfindung

Der vorliegenden Erfindung liegt die Aufgabe zugrunde, eine in-vivo-Darstellung und Quantifizierung oberflächlich lokalisierter fluoreszierender Markersubstanzen im Gewebe zu ermöglichen und insbesondere je nach Wellenlängenbereich verwendete Markersubstanzen in tiefer gelegenen Quellen zu lokalisieren und dort auch zu quantifizieren.

Das erfindungsgemäß vorgeschlagene Verfahren der Intravitaltomographie erweitert die klassische Intravitalmikroskopie um die Aspekte der Tiefenauflösung und der Farbstoffquantifizierung, insbesondere der Quantifizierung von fluoreszierenden Markersubstanzen im Gewebe. Bei der Intravitaltomographie handelt es sich um ein Quantitäten ermittelndes Verfahren, bei dem jedoch keine Durchleuchtung des gesamten Tieres von allen Seiten erforderlich ist, sondern eine Durchleuchtung lokaler beschränkter Gebiete angewendet werden kann. Die Intravialtomographie kann auch in Bereichen, die bisher aufgrund der limitierten Eindringtiefe des Lichts der intravitalen Mikroskopie vorbehalten waren, angewendet werden. Dies bezieht sich ebenso auf die Verwendung von Hautfenstern oder Hautklappen, die aufgrund der üblicherweise implantierten Fremdkörper, die das Zuwachsen oder die Heilung der entsprechenden Hautstelle verhindern sollen, für volltomographische Aufnahmen sehr hinderlich sind. Hautfenster und -klappen stellen die Stellen dar, an denen die Haut des Versuchstieres entfernt wurde, um die durch diese erzeugte Streuung und Schwächung zu beseitigen, um direkten Einblick unter das unter der Haut liegende Gewebe zu gewährleisten. Bei der Hautklappe wird dabei im einfachsten Fall die Haut vorübergehend umgeklappt und nach Begutachtung des darunter liegenden Gewebes wieder angenäht. Beim Fenster wird die Haut entfernt und durch einen eingelegten Ring wird verhindert, dass die zuvor freigelegte Stelle wieder zuwächst. Um eine Austrockung des Gewebes zu vermeiden, wird dabei meist ein glasklares luftdichtes Material auf diese Stelle aufgebracht. Fenster und Klappen werden hauptsächlich dazu eingesetzt, um die hohe Streuung, die Autofluoreszenz und die Schwächung durch die oberen Hautschichten zu eliminieren. Zudem sind für die Fluoreszenztomographie unbehaarte oder enthaarte Tiere nötig. Ist eine Hautklappe oder ein Hautfenster vorhanden, kann die Intravitaltomographie auch in diesem Bereich zur Ermittlung quantitativer und räumlich aufgelöster Informationen eingesetzt werden. Insbesondere lassen sich im Wege der Intravialtomographie Eindringtiefen bzw. Informationen über die Lage von fluoreszierenden Markersubstanzen im Gewebe in vivo und in situ erreichen.

Durch die Begrenzung auf ein kleines Gebiet kann im Wege der Intravitaltomographie eine höhere räumliche Auflösung im Vergleich zur Fluoreszenztomographie erreicht werden. Im Vergleich zur bekannten Konfokalmikroskopie wird die Auflösung wesentlich geringer sein, allerdings die Eindringtiefe signifikant höher. Die im Wege der Intravitaltomographie erhaltenen Informationen bzw. Bilder stellen demnach eine Zwischenstufe zwischen makroskopischer und mikroskopischer Fluoreszenzbildgebung dar.

Der erfindungsgemäß vorgeschlagenen Lösung folgend wird ein bekanntes Intravitalmikroskop mit optisch-tomographischen Techniken verknüpft. Die Abbildung eines aufgenommenen Bildes erfolgt über Strahlteiler durch Filter-Mechanismen eines Fluoreszenzmikroskopes, demnach durch eine Kombination von einem halbdurchlässigen Filterspiegel (dichroitischer Spiegel) und einem Emissionsfilter. Als Lichtquelle wird jedoch nicht unkollimiertes Licht zur flächigen Beleuchtung, sondern ein kollimierter, idealerweise monochromatischer oder schmalbandiger Lichtstrahl, wie beispielsweise durch einen Monochromator erzeugt, verwendet, der über den bildzugebenden Bereich gescannt werden kann. Die Detektion erfolgt nicht mit dem bloßen Auge, sondern entweder mittels einer hochempfindlichen, gekühlten Farb-CCD-Kamera oder mittels einer üblicherweise eingesetzten hochempfindlichen Schwarz/Weiß-CCD-Kamera und einem variablen Filter. Bei diesem kann es sich entweder um einen akustooptischen oder um einen Flüssigkristallfilter handeln, alternativ auch um eine Anordnung verschiedener Bandpassfilter in Form eines Filterrades.

Durch die Vornahme des Scans des Anregungsstrahles entstehen verschiedene detektierte Bilder. Jedes der detektierten Bilder enthält Informationen, die aus verschiedenen Gewebetiefen stammen. Bildpunkte (= Detektoren) mit einem größeren Abstand vom Einstrahlungspunkt erhalten ihre Intensität durch Photonen, die im Mittel einen tiefer gelegenen Weg durch das Gewebe zurückgelegt haben. Dies lässt sich Wahrscheinlichkeitsdichteverteilungen der Ausbreitungspfade entnehmen. So lassen sich Bildpunkte anhand ihres Abstandes zu gewählten Einstrahlungspunkten jeweils Ebenen, die im Gewebe in verschiedener Tiefe liegen, zuordnen. Der Anteil oberflächennäher gelegener Ebenen in Bezug zu den Ausbreitungspfaden durch tiefere Ebenen lässt sich subtraktiv aus den tieferen Ebenen eliminieren. Sind die Ausbreitungspfade für verschiedene Quell-Detektor-Abstände bekannt, ergibt sich ein einfaches lineares System, das zur Bestimmung der tatsächlichen Tiefe und damit auch zur Konzentrationsbestimmung von fluoreszierenden Markersubstanzen genutzt werden kann. Die Geometrie des Gewebes spielt aufgrund der Begrenzung auf ein kleines Areal eher eine untergeordnete Rolle.

Dieses Rekonstruktionsverfahren kann auch durch komplexere mathematische Konstruktionsverfahren sowie durch andere Verfahren, wie in der nachfolgend genannten Publikation beschrieben, durchgeführt werden: "Optical tomography in medical imaging", SR Arridge, Inv Prob 15: R41-R93, 1999. Dabei ist zu beachten, dass das mathematische Modell der Diffusionsgleichung in den hier in Betracht kommenden geringen Eindringtiefen und bei geringen vorliegenden Quelldetektorabständen nicht gilt und demzufolge durch ein anderes mathematisches Modell ersetzt werden muss. Dazu bieten sich die Strahlungstransportgleichung oder ähnliche Modelle an, die unter Umständen direkt gelöst werden können, die andererseits auch analytisch genähert werden können - soweit Näherungen verfügbar sind - oder die empirisch mittels Monte-Carlo-Simulation angenähert werden können. Im Rahmen einer Monte-Carlo-Simulation erfolgt die Simulation einer Lichtausbreitung einer kollimierten Punktquelle in ein einseitig unbeschränktes Medium. Eine Seite des Objektes wird beispielsweise als planar angenommen. Dieser Simulation liegt ein in Schichten aufgeteiltes Gewebemodell mit unterschiedlichen Schichteneigenschaften zugrunde. Eine berechnete Verteilungsfunktion kann aufgrund der Symmetrie entlang der x- und der y-Achse einfach verschoben werden (vorausgesetzt, die z-Richtung ist die Richtung, die in das Gewebe hineinweist). Durch die Verschiebung der Verteilungsfunktion kann die Quellverteilung zu einer beliebigen Quelle an der Oberfläche erhalten werden. Das Korrespondenzprinzip, wonach die Sensitivität eines Punktdetektors dafür, dass er Photonen aus einem bestimmten Gewebe detektiert, genau dem umgekehrten Prozess entspricht, zur Verteilung der Photonen aufgrund einer Quelleinstrahlung. Dadurch kann für die Detektorsensitivität diese eine berechnete Verteilung verwendet werden. Die sich daraus ergebenden bananenförmigen Kurvenverläufe ergeben sich durch einfache Multiplikation zweier solcher Verteilungsfunktionen.

Insbesondere für eine anvisierte Rekonstruktion fluoreszierender Quellen im sichtbaren und nahinfraroten Wellenlängenbereich spielt die Autofluoreszenz des Gewebes eine wichtige Rolle. Um den Effekt der Autofluoreszenz des Gewebes zu minimieren, wird im Rahmen der vorliegenden Erfindung eine spektrale Aufnahme vorgeschlagen, bei der für jeden Bildpunkt mehrere Aufnahme mit verschiedenen Emissionswellenlängen erfolgen, um so für jeden Punkt ein zumindest diskretes Spektrum der Emission zu erhalten, das mit dem Spektrum der verwendeten fluoreszierten Markersubstanz verglichen werden kann. Durch diese Technik kann die Autofluoreszenz des Gewebes und deren Verfälschungseinfluss weitestgehend minimiert werden.

### Kurze Beschreibung der Zeichnungen

Anhand der Zeichnungen wird die Erfindung nahe stehend eingehender beschrieben.

### Es zeigt:

- Figur 1: das Arbeitsprinzip einer quantitativ planaren Versuchseinheit zur Durchführung der Intravitaltomographie,
- Figur 2: eine beispielhafte Darstellung von Propagationspfaden von Photonen durch mit einer Anregung belegtes Gewebe,
- Figur 3: eine spektrale Information verschiedener fluoreszierender Substanzen, wobei es sich um Wasser, eine wässrige Lösung von Quantum Dots und eine Lösung von Cy3-Farbstoff handelt, und
- Figur 4: eine spektrale Information verschiedener fluoreszierender Substanzen, wie Wasser, eine wässrige Lösung von Quantum Dots und eine Lösung von Cy3-Farbstoff, aufgenommen mittels einer Schwarz/Weiß-Kamera.

### Bevorzugte Ausführungsformen der Erfindung

Der Darstellung gemäß Figur 1 ist eine Versuchseinheit zur Durchführung der Intravitaltomographie im prinzipiellen Aufbau zu entnehmen.

Die Versuchseinheit gemäß der Darstellung in Figur 1 umfasst ein Intravialmikroskop 10, welches mit einem Tubus 33 samt darin aufgenommener Einbauten oberhalb eines Objektes 12, wie zum Beispiel eines lebenden Tieres, in situ angeordnet werden kann. Mit Bezugszeichen 14 ist die Objektoberfläche bezeichnet, die im Falle eines als Tier beschaffenen Objektes dessen Haut oder rasierte oder enthaarte Hautstellen, eine Hautklappe oder dergleichen darstellt. Das zu untersuchende Objekt 12 ist auf einer Unterlage 16 aufgelegt oder auf dieser so fixiert, dass es seine Lage zum oberhalb des Objektes 12 angeordneten Intravitalmikroskop 10 während der Untersuchung nicht ändert.

Dem Intravitalmikroskop 10 ist eine Scaneinheit 18 zugeordnet. Die Scaneinheit 18 umfasst einen Anregungslaser 20. Als Anregungslaser wird eine Lichtquelle verwendet, die unkollimiertes Licht zur flächigen Beleuchtung, idealerweise monochromatisches Licht, in der Praxis schmalbandiges Licht, verwendet. Das schmalbandige Licht wird bevorzugt durch einen Monochromator erzeugt, der ohne Bandpassfilter auskommt. Mit der durch Bezugszeichen 34 angedeuteten Anregung kann in der Scaneinheit 18 innerhalb eines Bildbereiches 22 um einen Verfahrbereich 24 gescannt werden, so dass ein größerer Teil des Objektes 12 mit der Anregung 34 abgetastet werden kann. Wie aus der Darstellung gemäß Figur 1 hervorgeht, wird die Anregung 34 des Anregungslasers 20 über einen im Tubus 33 des Intravitalmikroskopes 10 geneigt angeordneten dichroitischen Spiegel 26 um eine erste Ablenkung, bevorzugt 90° abgelenkt und auf ein Objektiv, bei dem es sich bevorzugt um ein telezentrisches Objektiv 30 handelt, geworfen. Das Objektiv 30 ist unterhalb des dichroitischen Spiegels 26 angeordnet, auf dessen erste Spiegelseite 27 die vom Anregungslaser 20 erzeugte Anregung 34 fällt. Die Rückseite des dichroitischen Spiegels 26 ist durch Bezugszeichen 29 als zweite Spiegelseite bezeichnet.

In einer altvernativen Anordnung kann der Filter und die Einspeisung der Anregungsquelle zwischen Objektiv 30 und Objekt angeordnet sein, statt zwischen Objektiv 30 und der Kamera.

Die Anregung 34 gelangt durch das Objektiv 30, dessen Linsenfläche durch Bezugszeichen 32 angedeutet ist, in Richtung auf die Objektoberfläche 14 des Objektes 12, welches unterhalb des Objektivs 30 des Intravitalmikroskops 10 angeordnet ist.

Die vom Objekt 12 bzw. dessen Objektoberfläche 14 emittierte Strahlung gelangt im Rahmen eines Emissionsspektrums 36 nach Durchlaufen des Objektivs 30 und Durchlaufen der ersten Spiegelseite 27 sowie der zweiten Spiegelseite 29 des dichroitischen Spiegels 26 zum Emissionsfilter 38. Die dem dichroitischen Spiegel 26, genauer dessen zweiter Spiegelseite 29 zuweisende erste Seite des Emissionsfilters 38 ist durch Bezugszeichen 37, dessen zweite, einer CCD-Kamera 40 zuweisende Seite ist durch Bezugszeichen 39 bezeichnet. Im Emissionsfilter 38 wird das Emissionsspektrum zum Beispiel in eine Emission 1, Bezugszeichen 54, und eine Emission 2, Bezugszeichen 60, zerlegt und gelangt von dort in die CCD-Kamera 40. Bei der im Tubus 33 des Intravitalmikroskops aufgenommenen CCD-Kamera 40 kann es sich entweder um eine hochempfindliche, gekühlte Farb-CCD-Kamera oder um eine hochempfindliche Schwarz/Weiß-CCD-Kamera mit einem variablen Filter handeln. Bei dem variablen Filter, der der Schwarz/Weiß-CCD-Kamera bevorzugt vorgeschaltet ist, handelt es sich zum Beispiel um einen akustooptischen oder um einen Flüssigkristallfilter. Die CCD-Kamera 40, sei es eine Farb-CCD-Kamera, sei es eine Schwarz/Weiß-CCD-Kamera, stellt eine Ausführungsvariante eines optischen Flächendetektorelementes dar. Anstelle einer CCD-Kamera kann auch CMOS-Technik eingesetzt werden. Je nach Anwendungszweck können Filter für die Emissionsfilterung eingesetzt werden. Dabei kann es sich um die bereits vorstehend erwähnten Filterräder handeln, bei denen eine Vielzahl von Filterelementen untergebracht sein können, die je nach Einsatzzweck eingesetzt werden können. Wird eine breitbandige, aber kollimiertes Licht emittierende Quelle eingesetzt, ist ein Filter am Strahlgang der Anregungsquelle zu sehen, der vor oder hinter dem Scanelement angeordnet werden kann. Wird schmalbandiges Licht verwendet, so kann dieses mittels eines Monochromators erzeugt werden, unter Verzicht auf Bandpassfilter.

Der Darstellung gemäß Figur 2 gehen verschiedene, aus einer Anregung einer Oberfläche resultierende Propagationspfade der Emissionen durch ein Gewebe hervor.

Bei dem in Figur 2 dargestellten, vergrößert wiedergegebenen Ausschnitt von Gewebe 66 handelt es sich zum Beispiel um Gewebe eines lebenden Tieres, dessen Haut, sei sie enthaart, sei sie noch mit Haaren versehen, bzw. dessen Hautklappe mit einer Anregung 34 bestrahlt wird. Die Anregung 34 trifft auf der Objektoberfläche 14, 50 am Anregungspunkt 52 auf und dringt in tiefer liegende Hautschichten des Gewebes 66 ein. Bei dem Eindringen der Anregung 34 in das Gewebe 66 fährt der Strahl der Anregung 34, je nachdem auf welche Objekte er im Gewebe 66 trifft, per Strahlaufspaltung. Gemäß Figur 2 geht hervor, dass durch den Scan, d. h. das Verfahren des Anregungsstrahles 34 innerhalb des Verfahrbereiches 24, verschiedene detektierte Bilder entstehen. Das Verfahren des Anregungsstrahles 34 wird zum Beispiel mit handelsüblichen piezogesteuerten Spiegeln erreicht. Jedes dieser aufgenommenen Bilder enthält Informationen, die aus verschiedenen Tiefen des Gewebes 66 stammen. Bildpunkte mit einem größeren Abstand vom Anregungspunkt 52, d. h. des Einstrahlungspunkt in die Objektoberfläche 40, erhalten ihre Intensität durch Photonen, die im Mittel einen tiefer gelegenen Weg durch das Gewebe 66 zurückgelegt haben. Deren Ausbreitungspfade sind durch die in Bezug auf die Emission 1, Bezugszeichen 54, dargestellten ersten und zweiten Propagationspfade 56, 58 und in Bezug auf die Emission 2, vergleiche Bezugszeichen 60, durch den dritten und vierten Propagationspfad 62, 64 angedeutet. Die dargestellten Propagationspfade 56, 58; 62, 64 im Gewebe 66 entsprechen "bananenförmig" konfigurierten Wahrscheinlichkeitsdichteverteilungen der Photonen im Gewebe. Auf diese Weise lassen sich Bildpunkten anhand ihres Abstandes zu den gewählten Einstrahlungspunkten, d. h. Anregungspunkten 52, Ebenen verschiedener Tiefe zuordnen. In der Darstellung gemäß Figur 2 ist dem ersten Propagationspfad 56 eine erste Tiefe 68 entsprechend einer Ebene 1 zugeordnet. Der zweite, tiefer gelegen im Gewebe 66 verlaufende Propagationspfad 58 korreliert zur zweiten Tiefe 70, d. h. einer Ebene 2. In Bezug auf die Emission 2, vergleiche Bezugszeichen 60, verläuft im Gewebe 66 ein dritter Propagationspfad 62 in einer dritten Tiefe 72, entsprechend einer Ebene 3, und schließlich der vierte Propagationspfad 64 in einer vierten Tiefe 74, entsprechend einer Ebene 4, am tiefsten durch das zu untersuchende Gewebe 66. Der Anteil der oberflächennahe gelegenen Ebene in Bezug auf die Ausbreitungspfade durch tiefere Ebenen im Gewebe 66 lässt sich subtraktiv aus der tiefer gelegenen Ebene, in diesem Fall der Ebene 3, vergleiche Position 72, und der Ebene 4, vergleiche Position 74, gewinnen. Sind die Propagationspfade für verschiedene Quell-Detektor-Abstände bekannt, ergibt sich ein einfaches lineares System, das zur Bestimmung der tatsächlichen Tiefe und damit auch zur Bestimmung der Tiefe von Farbstoffkonzentrationen und fluoreszierender Markersubstanzen ausgenutzt werden kann.

Alternativ ist es möglich, anstelle einer einfachen Zuordnung von Quelldetektorabständen zu Ebenen, die emittierte und detektierte Strahlung direkt über die Wahrscheinlichkeitsdichtefunktion räumlich zu verteilen, so dass sich nicht einfache Ebenen, sondern die komplette Bananenform ergibt. Auch in diesem Zusammenhang entsteht ein lineares System.

Die Geometrie des Gewebes 66 spielt aufgrund der Begrenzung des innerhalb des Verfahrbereiches 34 verfahrbaren Anregungsstrahles 34 eine untergeordnete Rolle.

Das Signal muss nicht zwingend einer einzigen Ebene zugeordnet werden. Die Wahrscheinlichkeitsdichte bildet stattdessen ab, woher die Strahlung vorzugsweise stammt. Das lineare System entspricht in diesem Zusammenhang genau der Wahrscheinlichkeitsdichte. Für ein Quell-Detektorpaar 1 enthält ein Voxel 1 das Gewicht 1 - 1, das Voxel 2 enthält das Gewicht 2 - 1 usw. Für Paar 2, d. h. Quell-Detektorpaar 2, enthält das Voxel 1 das Gewicht 1 - 2 und das Voxel 2 das Gewicht 2 - 2. Das resultierende Gleichungssystem wird invertiert um die Lösung zu erhalten.

Falls erforderlich kann bei Anwendung der bekannten Methoden auch die Geometrie mit in die Berechnungen einbezogen werden. Dies entfällt in der Regel jedoch aus Geschwindigkeitsgründen, da die Wahrscheinlichkeitsdichtefunktionen für jedes Objekt neu berechnet werden müssten. Dies könnte jedoch wiederum deren Mehrfachnutzung ermöglichen.

Das obenstehend dargelegte Rekonstruktionsverfahren kann alternativ durch komplexere mathematische Rekonstruktionsverfahren durchgeführt werden. Dabei ist zu beachten, dass das mathematische Modell der Diffusionsgleichung in den betrachteten geringen Eindringtiefen und bei geringen Quell-Detektorabständen nicht gilt und durch ein anderes Modell zu ersetzen ist. Dazu bieten sich die Strahlungstransportgleichung oder ähnliche Modelle an, die unter Umständen direkt gelöst werden können, die analytisch genähert werden können - soweit Näherungen verfügbar sind - oder die emperisch mittels Monte-Carlo-Simulationen angenähert werden können. Insbesondere ist zu berücksichtigen, dass für die anvisierte Rekonstruktion fluoreszierender Quellen im sichtbaren bis nahinfraroten Wellenlängenbereich die Autofluoreszenz des Gewebes 66 eine wichtige Rolle spielt.

Die Zuordnung einzelner Ebenen, wie obenstehend beschrieben, benötigt ein Ausbreitungsmodell, um die Wahrscheinlichkeitsdichtefunktion und damit die Tiefen korrekt bestimmen zu können. Alternativ ist es möglich, anstelle einer einfachen Zuordnung von Quell-Detektor-Abständen zu Ebenen, die emittierte detektierte Strahlung direkt über die Wahrscheinlichkeitsdichtefunktion räumlich zu verteilen, so dass sich nicht einfache Ebenen, sondern die komplette Bananenform ergibt. Auch hier entsteht ein lineares Gleichungssystem. Bei den beiden bisher geschilderten Ansätzen unter der Brücksichtigung der Wahrscheinlichkeitsdichtefunktionen werden diese einmal berechnet und können beliebig oft wieder verwendet werden. Aus den Wahrscheinlichkeitsdichtefunktionen, die mit einem beliebig komplexen Modell gefunden werden können, ergibt sich stets ein lineares System. Es können jedoch auch nicht-lineare Systeme eingesetzt werden, dann fällt auch das erhaltene lineare System am Schluss weg und wird durch eine iterative Lösung ersetzt.

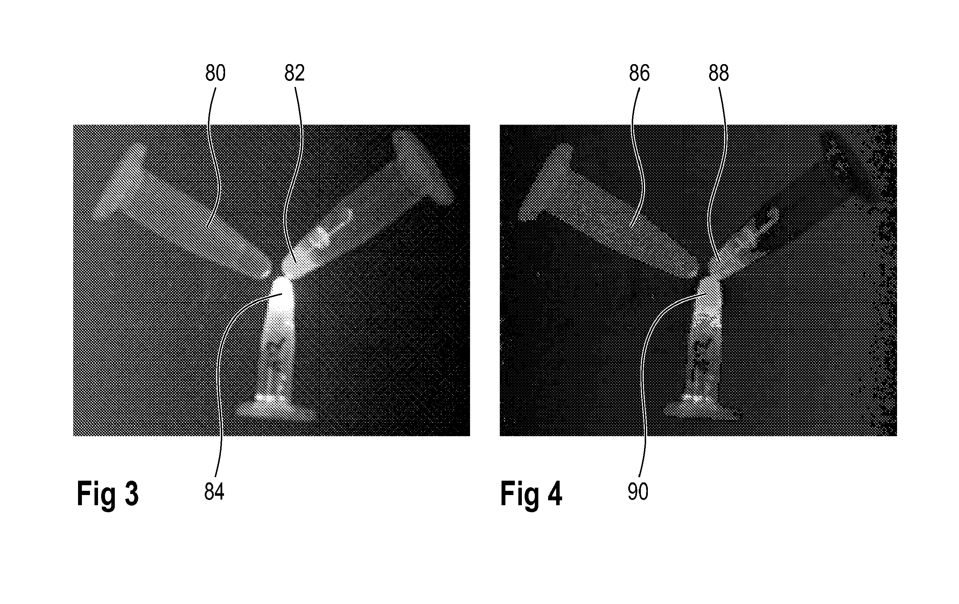

Um den Effekt der Autofluoreszenz zu minimieren, wird im Rahmen der vorliegenden Erfindung eine spektrale Aufnahme vorgeschlagen, bei der für jeden Bildpunkt mehrere Aufnahmen verschiedener Emissionswellenlängen erfolgen, um so für jeden Punkt ein Spektrum der Emission zu erhalten, das mit dem Spektrum der jeweiligen fluoreszierenden Markersubstanz verglichen werden kann. Dieses Verfahren lässt sich zum Beispiel mit einer gekühlten CCD-Kamera 40 verwirklichen, die im Tubus 33 des Intravitalmikroskops 10 angeordnet ist. Bei Einsatz einer gekühlten Farb-CCD-Kamera 40 können drei oder mehr verschiedene Kanäle, so zum Beispiel Rot, Grün und Blau gleichzeitig aufgenommen werden und eine klare Trennung verschiedener Farbstoffe bei gleicher Anregung erzielt werden. Vergleiche Darstellung gemäß Figur 3 und Gegenüberstellung mit Schwarz/Weiß-CCD-Aufnahme gemäß Figur 4.

Eine Alternative zum Einsatz einer gekühlten Farb-CCD-Kamera 40 liegt darin, einer hochempfindlichen Schwarz/Weiß-Kamera, bei der es sich auch um eine CCD-Kamera handeln kann, einen elektronisch steuerbaren Filter vorzuschalten. Über den elektronisch steuerbaren Filter kann die Einstellung der aufgenommenen Wellenlänge im Nanometerbereich erfolgen. Bei dem der CCD-Kamera vorzuschaltenden Filter, insbesondere eines elektronisch steuerbaren Filters, handelt es sich entweder um einen akustooptischen Filter oder einen flüssigkristallbasierten Lyot-Filter. Mit diesem elektronisch steuerbaren Filter können die Spektren an mehr als nur drei Punkten, so Rot, Grün und Blau, aufgenommen werden, so dass eine genauere aufgefächerte Analyse des Spektrums durchgeführt werden kann.

Der Darstellung gemäß Figur 3 ist die spektrale Information fluoreszierender Substanzen zu entnehmen.

Die Darstellung gemäß Figur 3 entspricht einer Aufnahme, die mittels einer gekühlten Farb-CCD-Kamera 40 durchgeführt wurde und drei Substanzen darstellt, die mittels blauem Licht angeregt wurden (Emission bei 705 nm): Wasser, angedeutet durch Bezugszeichen 80, wässrige Lösung von Quantum Dots, vergleiche Bezugszeichen 82, sowie eine Lösung des Cy3-Farbstoffes, vergleiche Position 84. Aus der Darstellung gemäß Figur 3 geht hervor, dass die Farbstoffe eindeutig voneinander unterscheidbar sind. Der Darstellung gemäß Figur 4 ist demgegenüber eine Aufnahme mit einer Schwarz/Weiß-Kamera zu entnehmen. Bei der in Figur 4 dargestellten spektralen Information handelt es sich um die Aufnahme fluoreszierender Substanzen, die ebenfalls mittels blauem Licht einer Wellenlänge von 750 nm angeregt wurden. Dabei handelt es sich im Einzelnen um H₂O, vergleiche Position 86, eine wässrige Lösung von Quantum Dots, vergleiche Position 88, sowie um angeregten Cy3-Farbstoff, vergleiche Position 90 in Figur 4.

Das erfindungsgemäß vorgeschlagene Bildgebungsverfahren der Intravitaltomographie wird bevorzugt mit einem Intravitalmikroskop 10 durchgeführt, welches einen Filterblock umfasst, der zumindest den Emissionsfilter 38 und den geneigt im Tubus 33 des Intravitalmikroskops 10 angeordneten dichroitischen Spiegels 26 enthält. Der dargestellte Filterblock erlaubt es, die Anregung 34, austretend aus der Scaneinheit 18, umgelenkt entsprechend der ersten Umlenkung 28 an der ersten Spiegelseite 27 des dichroitischen Filters 26, und die Detektion des Emissionsspektrums 36 über ein und dasselbe Objektiv, d. h. in diesem Falle die telezentrische Linse 30, vorzunehmen. Das Objektiv 30 kann unter anderem zum Beispiel eine telezentrische Linse umfassen, durch welche eine Vergrößerung des Bildbereiches erzielt werden kann. Telezentrische Linsen liefern unabhängig vom Abstand die gleiche Größe. Des Weiteren weisen telezentrische Linsen den Vorteil auf, dass diese einen wohl definierten Strahlengang erzeugen.

In Bezug auf die Darstellung gemäß Figur 2 sei angemerkt, dass die detektierten Werte Informationen über Geweberegionen des Gewebes 66 enthalten, abhängig vom Abstand zwischen dem jeweiligen Detektionspunkt, vergleiche erste Tiefe 68, zweite Tiefe 70, dritte Tiefe 72 und vierte Tiefe 74, und dem betreffenden Einstrahlpunkt 52 der Anregung 34 tiefer oder höher liegen. In der Darstellung gemäß Figur 2 ist dies angedeutet durch die bananenförmig verlaufenden, durchschnittlichen Propagationspfade, vergleiche Positionen 56 und 58, in Bezug auf die Emission 1, Bezugszeichen 54, und vergleiche dritten und vierten Propagationspfad 62, 64 in Bezug auf die Emission 2, vergleiche Bezugszeichen 60.

Wie aus der Darstellung der Figur 2 des Weiteren hervorgeht, stellen die Bildpunkte C und D, in denen die Emissionen 1, 2, vergleiche Bezugszeichen 54, 60, gemessen werden, die Detektionspunkte dar. Als Quelle der Anregung 34 gilt der Einstrahlungspunkt 52, an dem die Anregung 34 eingestrahlt wird. Der sich ergebende Quelldetektorabstand für die Detektion am Bildpunkt C ist in der Darstellung gemäß Figur 2 mit A bezeichnet, der Quelldetektorabstand für den Bildpunkt D ist in Figur 2 durch den Abstand B bezeichnet.

### Bezugszeichenliste

- 10: Intravitalmikroskop
- 12: Objekt
- 14: Objektoberfläche (Haut/Hautklappe)
- 16: Unterlage
- 18: Scaneinheit
- 20: Anregungslaser
- 22: Bildbereich
- 24: Verfahrbereich
- 26: dichroitischer Spiegel
- 27: erste Spiegelseite
- 28: erste Ablenkung 90°
- 29: zweite Spiegelseite
- 30: Objektiv
- 32: Linsenfläche
- 33: Tubus
- 34: Anregung
- 36: Emissionsspektrum
- 37: erste Seite
- 38: Emissionsfeld
- 39: zweite Seite
- 40: optisches Flächendetektorelement (CCD)
- 50: Oberfläche
- 52: Einstrahlungspunkt
- 54: Emission 1
- 56: erster Propagationspfad Emission 1
- 58: zweiter Propagationspfad Emission 1
- 60: Emission 2
- 62: dritter Propagationspfad Emission 2
- 64: vierter Propagationspfad Emission 2
- 66: Gewebe
- 68: erste Tiefe (Lage Ebene 1)
- 70: zweite Tiefe (Lage Ebene 2)
- 72: dritte Tiefe (Lage Ebene 3)
- 74: vierte Tiefe (Lage Ebene 4)
- 80: angeregtes H₂O (Farbe)
- 82: angeregte wässrige Lösung (Farbe)
- 84: angeregter Cy3-Farbstoff (Farbe)
- 86: angeregtes H₂O Schwarz/Weiß-Aufnahme)
- 88: angeregte wässrige Lösung Schwarz/Weiß Aufnahme
- 90: angeregter Cy3-Farbstoff Schwarz/Weiß

## Patentansprüche

1. Bildgebungsverfahren zur Darstellung beschränkter Ausschnitte eines Gewebes (66) zur Tiefenauflösung und zur Quantifizierung fluoreszierender Markersubstanzen im Gewebe (66) mit nachfolgenden Verfahrensschritten:
a) Als Anregungsquelle (20) wird ein bevorzugt monochromatischer Lichtstrahl verwendet, der über einen dichromatischen Spiegel (26) auf das Objekt (12) gerichtet wird,
b) der bevorzugt monochromatische Lichtstrahl wird innerhalb eines Scanbereiches (22)/Verfahrbereiches (24) relativ zum Objekt (12) verfahren, wobei detektierte Bilder verschiedener Tiefen (68, 70, 72, 74) aufgenommen werden, und
c) die Detektion von Emissionen (36; 54, 60) erfolgt nach Passage eines Emissionsfilters (38) durch eine CCD-Kamera (40).

2. Bildgebungsverfahren gemäß Anspruch 1, **dadurch gekennzeichnet, dass** gemäß Verfahrensschritt b) erhaltene Bildpunkte mit einem größeren Abstand vom Einstrahlungspunkt (52) eine Intensität aufweisen, die von Photonen erzeugt wird, die im Mittel einen in einer größeren Tiefe (68, 70, 72, 74) gelegenen Propagationspfad (56, 58, 62, 64) durch das Gewebe (66) zurücklegen.

3. Bildgebungsverfahren gemäß Anspruch 1, **dadurch gekennzeichnet, dass** die an Bildpunkten C, D ermittelte Intensität austretenden Lichts (54, 60) anhand von Abständen A, B der Bildpunkte C, D zum Einstrahlungspunkt (54) der Anregung (34) Ebenen in unterschiedlichen Tiefen (68, 70; 72, 74) zugeordnet werden.

4. Bildgebungsverfahren gemäß Anspruch 1, **dadurch gekennzeichnet, dass** der Anteil oberflächennah gelegener Ebenen (68, 70) zu Propagationspfaden durch tiefere Ebenen subtraktiv aus den tieferen Ebenen bestimmt wird.

5. Bildgebungsverfahren gemäß Anspruch 1, **dadurch gekennzeichnet, dass** aus Propagationspfaden (56, 58; 64, 66) für verschiedene Quell-Detektor-Abstände tatsächliche Tiefen (68, 70; 72, 74) und Konzentrationen von fluoreszierenden Markersubstanzen ermittelt werden.

6. Bildgebungsverfahren gemäß Anspruch 1, **dadurch gekennzeichnet, dass** gemäß Verfahrensschritt c) die Detektion von Emissionen 1, 2 (54, 60) über eine gekühlte Farb-CCD-Kamera (40) erfolgt.

7. Bildgebungsverfahren gemäß Anspruch 6, **dadurch gekennzeichnet, dass** die gekühlte CCD-Kamera (40) mindestens drei verschiedene Kanäle aufweist, die gleichzeitige Aufnahme ermöglichen und eine Trennung verschiedener Farbstoffe bei gleicher Anregung (34) ermöglichen.

8. Bildgebungsverfahren gemäß Anspruch 1, **dadurch gekennzeichnet, dass** gemäß Verfahrensschritt c) die Detektion von Emissionen 1, 2 (54, 60) über einen elektronisch steuerbaren Filter erfolgt, der einer hochempfindlichen Schwarz/Weiß-CCD-Kamera vorgeschaltet ist.

9. Bildgebungsverfahren gemäß Anspruch 8, **dadurch gekennzeichnet, dass** als elektronisch steuerbarer Filter ein akustooptischer Filter oder ein flüssigkristallbasierter Filter verwendet wird, mit dem die Einstellung einer Wellenlänge im Nanometerbereich vorgenommen wird.

10. Bildgebungsverfahren gemäß Anspruch 1, **dadurch gekennzeichnet, dass** zur Minimierung der Autofluoreszenz des Gewebes (66) eine spektrale Aufnahme erfolgt, bei der für jeden Bildpunkt mehrere Aufnahmen verschiedener Emissionswellenlängen durchgeführt werden.

11. Bildgebungsverfahren gemäß Anspruch 10, **dadurch gekennzeichnet, dass** für jeden Bildpunkt ein Spektrum erhalten wird, welches mit dem Spektrum der mindestens einen fluoreszierenden Markersubstanz verglichen wird.

12. Bildgebungsverfahren gemäß Anspruch 1, **dadurch gekennzeichnet, dass** ein Intravitalmikroskop (10) eingesetzt wird, welches ein mit einer Neigung angeordneten dichromatischen Spiegel (26) aufweist, in das die Anregung (34) über die Scan-Einheit (18) eingekoppelt wird.

13. Bildgebungsverfahren gemäß Anspruch 12, **dadurch gekennzeichnet, dass** die Anregung (34) und die Detektion des Emissionsspektrums (36) über ein und dasselbe Objektiv (32), insbesondere eine telezentrische Linse erfolgen.

14. Bildgebungsverfahren gemäß Anspruch 1, **dadurch gekennzeichnet, dass** gemäß Verfahrensschritt b) die Anregung (34) mit der Scan-Einheit (18) über den gesamten Bildbereich (22) bewegt wird.

15. Verwendung eines Intravitalmikroskops (10) zur Durchführung des Bildgebungsverfahrens gemäß einem oder mehreren der Ansprüche 1 bis 14.

16. Verwendung gemäß Anspruch 15, **dadurch gekennzeichnet, dass** das Intravitalmikroskop (10) einen Filterblock aufweist, der einen dichromatischen Spiegel (26) und einen Emissionsfilter (38) umfasst.
